# EUROPEAN PATENT APPLICATION

(11) **EP 0 712 826 A1**
(43) Date of publication of application: **22.05.1996**
(21) Application number: 95113522.7
(22) Date of filing: 29.08.1995
(51) Int. Cl.: C07C 17/20, C07C 19/08

(54) **1,1,1-Trifluoroethane synthesis using a supported lewis acid**

(30) Priority: 17.11.1994 US 341621
(71) Applicant: ELF ATOCHEM NORTH AMERICA, INC., Philadelphia Pennsylvania 19102-3222 (US)
(72) Inventor: Elsheikh, Maher Yousef, Wayne, PA 19087 (US); Bolmer, Michael Sheppard, Collegeville, PA 19426 (US)
(74) Representative: Kraus, Walter, Dr.

(57) **Abstract**

Process for synthesizing 1,1,1-trifluoroethane (143a) in the gaseous phase by reacting 1,1-difluoro-1-chloroethane in gaseous phase in the presence of a catalyst selected from the SbV, TiIV,and SnIV salts. The process may be run without co-feeding chlorine gas as a coactivator, in the presence or absence of an impurity selected from HCl and 141b. The catalyst may be unsupported or supported on activated carbon. The formation of olefin byproduct was not detected when the process of the invention was conducted under the conditions described.

## Description

### FIELD OF THE INVENTION

The present invention relates to an improved method for producing 143a (1,1,1-trifluoroethane) by continuous gas phase hydrofluorination of 1,1-difluoro-1-chloroethane using heterogeneous catalysis.

### BACKGROUND OF THE INVENTION

1,1,1-Trifluoroethane (143a) is a hydrofluorocarbon (HFC) with zero ozone depletion potential (ODP). The product was described earlier in the literature as an undesirable co-product from processes involving hydrofluorination of 1,1 - dichloroethylene (VDC, 1130a) or 1,1,1-trichloroethane (140a). While methods are known for synthesizing 143a, there is a need for a simple, convenient, economical, industrial process for the manufacturing of 143a. The present invention provides a new practical process for the production of 143a with high yield.

In U.S. Patent No. 3,231,519, issued January 28, 1966 and assigned to Union Carbide Corporation, a catalyst composed of co-precipitated iron hydroxide and rare earth oxide, such as dysprosium hydroxide, and zirconium oxide was used to hydrofluorinate (140a) to a mixture of 143a, 142b and 1130a. Thus, when hydrogen fluoride (177 g, 8.85 moles) and 1,1,1-trichloroethane (541 g, 4.05 moles) were vaporized over 150 milliliters of the catalyst over a three to four hour reaction period at a temperature of 230-260°C, to give 1,1,1-trifluoroethane (122 g, 1.45 moles); 1-chloro-1,1-difluoroethane (33 grams, 0.328 moles); 1,1-dichloroethylene (157 g, 1.62 moles) and a small amount of 1-chloro-1-fluoroethylene. The latter two products, 1130a and 1131a, are a waste co-product; conversion was 83.9% and selectivity for 143a was 42.67% under these conditions. Catalysts claimed in this patent are a combination of iron oxide, rare earth oxide, and zirconium oxide. The lifetime of the catalyst was not reported.

U.S. Patent No. 3,287,424, issued November 22, 1986 and assigned to Stauffer Chemical Company, discloses the hydrofluorination of 1,1,1-trichloroethane (140a) to 1,1,1-trifluoroethane (143a) in a batch process, using arsenic trifluoride as a fluorinating agent and antimony pentafluoride as a catalyst. In Example 3, a mixture of arsenic trifluoride (333.25 grams, 2.53 moles) and antimony pentafluoride (29.9 grams, 0.14 moles) was reacted with methylchloroform (133 g, 1 mole) at 45-50°C to produce 1,1,1-trifluoroethane (63 g, 0.75 moles). The fluorinating agent, AsF₃, is a highly toxic material and is an expensive reagent for industrial applications.

U.S. Patent No. 3,803,241, assigned to Dynamit Nobel AG, uses a catalyst composed of chromium (III) chloride supported on alumina, prepared by soaking aluminum oxide pellets in CrCl₃·6H₂O solution (31 wt.%). The catalyst was dried at 200°C using nitrogen or air, followed by HF activation at 250°C for 2 hours. In Example 1, following the HF activation, a gaseous stream of 1,1-dichloroethylene and hydrogen fluoride in a molar ratio of 1:3.5 at 150°C was passed over the catalyst bed at 150°C, to yield 98.8 volume % of 1,1,1-trifluoroethane, 0.2 volume % of l42b, 0.2 volume % of 141b and 0.8 volume % of 1,1-dichloroethylene. After running for quite some time (exact running time not reported), the catalyst was regenerated by heating for 10-15 days. No experimental details were provided on how the catalyst was reactivated nor was there evidence that the catalyst performance improved after the treatment. Although the selectivity and conversion were very high, the catalyst required a very long time for regeneration, which is not practical for industrial applications.

In U.S. Patent No. 3,833,676, it is disclosed that hydrofluorination of methyl chloroform in a liquid phase batch process can produce very low levels of 1,1,1-trifluoroethane (Example 2). In this example, methyl chloroform (3.73 grams) and hydrogen fluoride (17 g) (molar ratio of HF:methyl chloroform = 30.3:1) were mixed together in a stainless steel reactor at 110°C for 2 hours to produce 2.3 mole % of 141b, 95.5 mole % of 142b and 2.1 mole % of 143a. This process is a liquid phase process and requires very long contact time, which means that it is much less productive compared to continuous gas phase processes.

In U.S. Patent No. 3,836,479, Example 1, a catalyst composed of boric acid (0.18 kg) mixed with pseudoboehmite alumina (1.2 kg) was prepared and activated using hydrogen fluoride at 350°C using 2 mole/hr HF and 1 mole/hr nitrogen. After the catalyst was activated, a mixture of HF (.75 mole/hr) and vinylidene fluoride (feed rate not reported) was passed over the catalyst at room temperature to produce 100% conversion to 143a. (Example 12) The feed stock of this process, 1,1-difluoroethylene, is an expensive compound for industrial application, and it is expected that 143a produced using this process will be expensive.

A bismuth containing catalyst supported on alumina was prepared in Example 1 of U.S. Patent No. 3,904,701 by soaking alpha-alumina (650 g) in a mannitol solution of Bi(NO₃)₃·5H₂0 (153 g). The catalyst was dried at 80°C for one hour. Subsequently it was activated at 250°C using a mixture of HF and air. Then a gaseous mixture of 1 part dichloroethylene and 3.2 parts of HF (Example 1) was passed over the catalyst bed at 180°C, with 18 seconds contact time. Analysis of the product obtained indicated that conversion was 99.9%; selectivity for 143a was 99.8% and for l42b it was 0.2%. In all the examples reported in this patent, halogenated alkenes were used as the feed stock. E.g., in Examples 1, 3, 4 and 5; 1,1-dichloroalkene was used as the starting material; in Example 2, vinyl fluoride monomer was used as the organic substrate. The composition of the catalyst of this patent (Bi/Al₂0₃) is totally different from that of the catalyst of the present invention. This patent also discloses an improved regeneration process for the above catalyst, by heating the deactivated catalyst in air at a temperature of about 350-450°C. This regeneration process is claimed in related U.S. Patent No. 3,965,038.

A continuous liquid phase process for the hydrofluorination of methylchloroform to the mixture of products 141b, 142b and 143a is disclosed in U.S. Patent No. 4,091,043. The process requires continuous feed of antimony pentachloride in the presence of organic solvent. This will require additional separation equipment to separate the antimony catalyst and the organic solvent, which is troublesome on the industrial scale. The best result for CH₃CF₃ selectivity (61.2%) was obtained when the reactor was initially charged with SbCl₅ (52.2 mole%) and 0.76 moles of the solvent 1,1,2-trifluoro-1,2,2-trichloroethane. The feed rate of methylchloroform was 0.76 mole/hour; for HF it was 2.32 mole/hour. At 28°C, conversion was 93%, while selectivity for 143a was 82.6%. Selectivity was 17.1% for 142b and 0.3% for 141b. A similar process was described in Atochem S.A.'s European Patent Publication No. 0 421 830 A1, which uses a combination of SbF₅ and chlorine gas as a catalyst for a HF/methylchloroform process. The percent selectivity of 143a varied between 1% to 10.3%, depending on the processing conditions. Again, this process requires recovery of the antimony catalyst. In the absence of chlorine gas, the active catalytic species, Sb(V), was reduced to the inactive catalyst species, Sb(III).

In U.S. Patent No. 4,147,733, Example 2, a catalyst composed of alumina coated with 12 percent by weight of Cr₂0₃ and 6% of Ni0, was used to hydrofluorinate chlorinated aliphatic hydrocarbons to the corresponding fluoride using aqueous HF, e.g. at 420°C. Feeding a mixture of 38% aqueous HF and 1,1-dichloroethylene vapors at a 3:1 molar ratio of HF/VDC, gave a total conversion of 16.3% to fluorinated product. The selectivity for l43a was 54.1 mole %, while it was 21% for l-chloro-1-fluoroethylene and 20.4% for vinylidene fluoride. This process requires the use of aqueous HF as a feed stock, which is known to be very corrosive compared to anhydrous HF gas. Furthermore, the presence of the fluoroolefin as impurity in l43a is undesirable for either refrigerant applications or foam blowing agent applications.

1,1,1-Trifluoroethane was also reported as a major co-product, during the fluorination of vinylidene fluoride, using activated carbon, in U.S. Patent No. 4,937,398. The process was directed towards the preparation of 1,1,1,2-tetrafluoroethane. Instead, 143a was the major product. The latter product was suggested to be obtained from a process involving HF addition to vinylidene fluoride. HF was disclosed to be generated by hydrolysis of fluorine gas by the moisture on the surface of activated carbon, e.g., when VF₂ (8 ccm) mixed with nitrogen (50 ccm) was slowly fed over activated carbon (40 grams, saturated with 6 wt% of fluorine gas). At 50°C, conversion was 100% and selectivity for 143a was 82%. Selectivity for 1,1,1,2-tetrafluoroethane (134a) was 18%. The implementation of this process for the production of 143a can be a very difficult task, because fluorine gas addition to olefin is a highly exothermic process.

In U.S. Patent No. 5,008,474, Example 1, hydrofluorination of 1,1-dichloroethylene in the presence of tin tetrachloride as a catalyst, in a batch liquid phase process, produced 143a in small quantities. E.g., when 5.16 moles of 1,1-dichloroethylene, 16.05 moles of HF and 0.25 moles of SnCl₄, were mixed together under continuous stirring, analysis of the product formed showed the following composition: 143a (2.1 mole %), 142b (26.7 %), 141b (64.8 %), vinylidene chloride (4.1 %), 1,1,1-trichloroethane (0.8%) and oligomeric material (1.4%). In Examples 2-4, the yields of 143a were even lower. Thus, the yield of 143a from this process is not high enough for it to be utilized as an industrial process.

The prior art describes processes that produce 143a which are based on hydrofluorinating either 140a or 1130a. The first compound (140a) is expected to be regulated by the U.S. federal government in the near future. The second compound (1130a) is known to undergo cationic polymerization to produce low molecular weight polymer and thereby deactivate the catalyst. (See McBeth et al., J. Chem. Soc., Dalton Trans., (1990) 671.) There is need for a simple, convenient and economical process for the production of 143a that avoids the foregoing problems.

### SUMMARY OF THE INVENTION

This invention provides a novel process for manufacturing 143a in an economical, industrially feasible manner, which is based on continuous gas phase hydrofluorination using heterogeneous catalysis. The organic feed is 1,1-difluoro-l-chloroethane (142b) and the fluorinating agent is HF. In the absence of catalyst, treating 142b with HF at 140°C, using a 3 molar ratio of HF/142b and 47 seconds of contact time, gave no conversion.

The catalyst used in the process of the present invention is an antimony V, tin IV or titanium IV salt, unsupported or supported on activated carbon. The catalysts ran continuously for hundreds of hours without any evidence of catalyst deactivation. Conversion was very high, depending on the operating conditions. This is particularly unexpected because of what is known about Lewis acid catalyzed hydrofluorination, e.g. in order to maintain the SbV catalyst activity, chlorine gas has to be continuously fed to maintain the catalyst activity, e.g. in the hydrofluorination of CH₂Cl₂ to CH₂F₂. See U.S. Patent No. 2,749,374. In the absence of chlorine gas, SbV is thermally reduced to SbIII with elimination of chlorine gas (SbF₃Cl₂-->SbF₃+Cl₂). The latter is known to be catalytically inactive in the hydrofluorination process. W. A. Sheppard and C. M. Sharts, Organic Fluorine Chemistry; W. A. Benjamin Inc. 80 (1969). In the process of this invention, stabilization of SbCl₅ may result from immobilization of Sb(V) by reacting the metal chloride with the hydroxyl groups present on the surface of activated carbon as shown below. 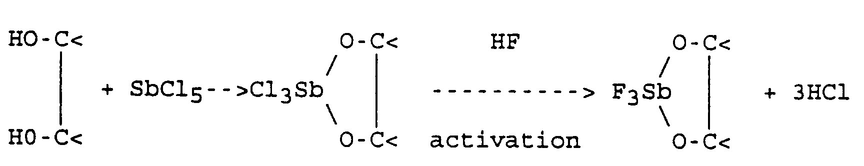
A similar approach was used for anchoring TiCl₄ and SnCl₄ on activated carbon surface.

The process of this invention does not require co-feeding of chlorine gas or other oxidizing agents to maintain the catalyst activity. Being able to avoid co-feeding of chlorine gas provides an important advantage because presence of the chlorine gas could result in the chlorination of the 143a product (CH₃CF₃) to unwanted chlorinated derivatives (CH₂ClCF₃, CHCl₂CF₃, and CCl₃CF₃).

### BRIEF DESCRIPTION OF THE FIGURES

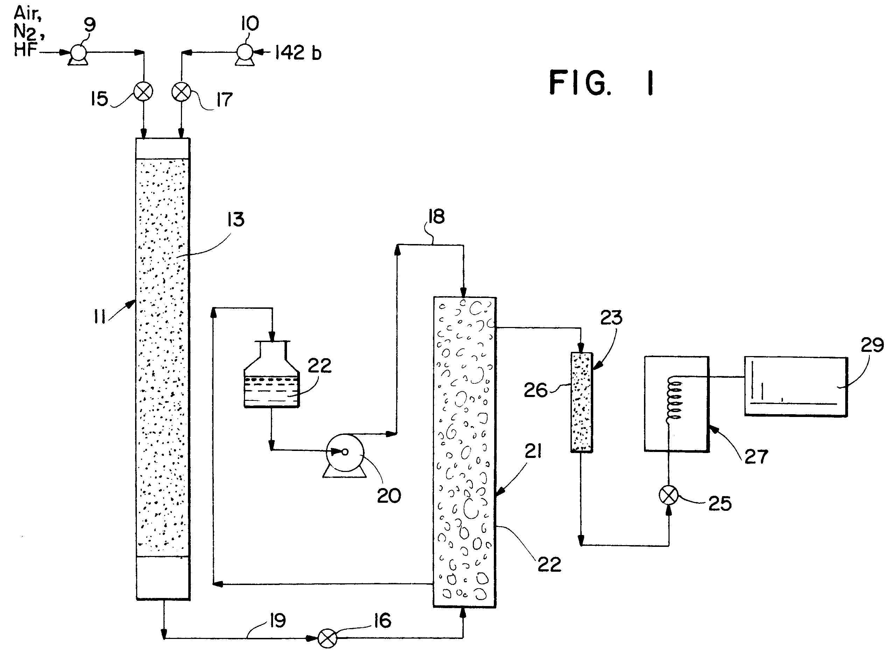

Figure 1 is a schematic diagram of a reactor suitable for carrying out the process of the invention.

### DETAILED DESCRIPTION OF THE INVENTION

Figure 1 illustrates a presently preferred reactor for carrying out the process of the present invention for preparing 143a by hydrofluorinating 142b. This reactor will be described in greater detail below in connection with Example 1.

In the process of this invention, 142b and HF are passed through the catalyst bed in a reactor at the specified conditions for reacting, and then the 143a product is purified. Byproduct HCl and unreacted HF may be removed by any number of methods known to the art, such as absorption in water or caustic solution or on solid absorbants, distillation, or membrane separation. Any unreacted 142b or byproduct 141b or olefins (1,1-difluoroethylene, 1,1-chlorofluoroethylene, 1,1-dichloroethylene) can also be removed, e.g., by distillation, absorption in either liquids or solids, or membrane separation.

Any olefins produced in the first reactor can be reacted with HF in a second reactor operating at a lower temperature. The thermodynamic equilibrium between olefins and saturated compounds strongly favors saturated compounds at lower temperatures. This configuration makes it possible to use a lower HF:142b molar ratio, such that olefins are produced in the first reactor and converted in the second reactor. This would minimize the amount of unreacted HF that would have to be neutralized or recycled. Unreacted excess HF can be separated and recycled to the first reactor.

The feed source can be pure 142b and HF or other streams containing these two compounds. 142b can be made by the reaction of HF with either 140a or 1130a. The product of this reaction will usually contain unreacted HF and HCl, as well as 141b byproducts. The use of unpurified feed streams containing 141b and HCl is illustrated in Example 4 below.

The reactor can be any vessel that allows the contact of the reactants with the catalyst for sufficient time to achieve the desired conversion. Materials of construction should be able to withstand HF and HCl at reaction temperatures, which are known to those skilled in the art. A plug flow reactor is preferred over a mixed reactor, such as a fluidized bed, in order to achieve high conversion in an efficient manner. The reactor can be cooled or not cooled, as long as the proper reaction conditions are maintained.

As noted above, the process can be carried out using 142b as a feed and 1,1-dichloro-1-fluoroethane (141b) or HCl as a co-feed. The process can be carried out at a temperature between 30°C and 250°C, preferably between 50°C and 160°C. Contact time can be varied from 1 to 100 seconds, preferably from 1 to 60 seconds and is more preferably between about 5 and about 50 seconds or, even more preferably, between 5 and 15 seconds. The catalyst has to be activated first using nitrogen, followed by a mixture of HF and nitrogen, at a temperature between 30 and 150°C, preferably between 50 and 100°C. Hydrofluorination can be performed at a pressure between 1 atmosphere and 200 psig, preferably between 1 atmosphere and 150 psig. The molar ratio of HF:142b can vary between 1:1 and 20:1. Preferably, it is between 1:1 and 10:1, more preferably between 2:1 and 5:1.

The catalyst used in the process of the present invention is selected from the salts of antimony V, tin IV and titanium IV. The catalyst may be unsupported or supported. In a preferred embodiment, it is supported on activated carbon. More particularly, presently preferred catalyst salts for use in the process of this invention are SbC1₅, TiCl₄, and SnC1₄, and other pentavalent antimony or tetravalent titanium or tin salts, which, when activated, are converted to the corresponding fluorides. We presently prefer to use SbCl₅, supported on activated carbon, as illustrated in the following examples.

In the following examples, unless otherwise indicated, the procedures are conducted under atmospheric pressure.

Example 1: Preparation and testing of catalysts: Sb/C, Ti/C, Sn/C.

The following general procedure was used to prepare .0017 mol. metal chloride/g of the catalyst. To preheated, pre-evacuated (130°C for four hours) activated carbon (200 grams, Calgon CPG) was added SbCl₅ (95 ml; 0.75 moles) dropwise over a one hour period with continuous shaking at 100°C. After completing the additions, the catalyst appeared to be very dry. The supported catalyst thus obtained was kept under anhydrous conditions. The fresh catalyst 13 (Sb/C) (57.2 grams) was loaded to a 3/4 inch (inner diameter) by 12 inch Hastelloy C reactor, 11, shown in Fig. 1. The catalyst was activated for 2 hours at 50°C, using 10 cc/m of nitrogen from valve 15, followed by HF activation at 100°C using 20 cc/m HF from valve 15. A total of 19.3 grams of HF was fed in 18 hours. The catalyst was evaluated at 140°C using a feed of 142b (20 cc/m) from valve 17 and HF (60 cc/m). (1 cc/m HF = 0.89 mg/m [milligrams per minute] of HF; 1 cc/m 142b = 4.48 mg/m of 142b.) The reaction products were removed at the bottom of reactor 11 through line 19 and backpressure regulator 16, and were then passed up through scrubbing tower 21, counter current to a stream 22 of alkaline solution, for example, 1-5 normal aqueous potassium hydroxide, which was circulated through line 18 by pump 20, to remove unreacted HF. Alternatively, the HF can be removed by distillation or other methods known in the art. Other aqueous hydroxides, such as sodium or calcium hydroxide suspension, can also be used as the alkaline solution. The product obtained was then passed through a drying tower 23, packed with a drying agent 26, such as anhydrous calcium sulfate. The conversion was periodically checked by passing product automatically through valve 25 to a gas chromatograph 27 equipped with electronic integrator 29. In the apparatus of Fig. 1, pumps 9 and 10 and a backpressure regulator at 16 facilitate operations of the apparatus at higher pressures, e.g. in excess of 100 psig.

Gas chromatograph (G.C.) analysis showed a steady 100% conversion and 100% selectivity for 143a. After running for approximately 500 hours, there was no evidence of catalyst deactivation or deterioration of the Sb/C catalyst performance, as shown in Table 1.

The same processing conditions were repeated using Sn/C and Ti/C catalyst, respectively, prepared as above but using 54.8 ml (0.47 moles) of TiCl₄ and 91.3 ml (0.78 moles) of SnCl₄, respectively. Conversion for the Sn/C was 91.1 %, and was 84.7% for the Ti/C. Upon increasing the temperature to 160°C (for Ti/C), conversion increased to 99.2%. In all three cases, selectivity for 143a was 100%, as shown in Table 1. It is clear from the data that the type of metal in the catalyst has a pronounced effect on the conversion. Sb (V) is the most active metal compared to Sn (IV) and Ti (IV). It is also clear that catalyst activity remains the same, without the need to co-feed chlorine.

Example 2: Effect of contact time and molar ratio of HF/142b on Sb/C catalyst performance.

The Sb/C catalyst (50.6 grams), prepared and activated as described in Example 1, was loaded to reactor 11. Then HF and l42b were fed at 100°C using various molar ratios (m.r.) of HF/142b (1.15 - 2.00) and various contact times (26 - 32 seconds). A summary of the results is shown in Table 2. After 85 hours of continuous running, conversion was still very high (>99.9%) and selectivity for l43a was 100%. In a separate experiment (also summarized in Table 2), the Sb/C catalyst (16.6 grams) was evaluated at 100°C, contact time 15 seconds using 1.1:1 molar ratio. After 336 hours of running, conversion was 99.6% and selectivity was 100%. The fact that catalyst activity was very high and unchanged during the 336 hours is an indication that the SbV catalyst was stable during the entire operation.

Example 3: Effect of metal concentration on the performance of the catalyst.

The effect of antimony concentration on the performance of Sb/C, and the effect of titanium level on the performance of Ti/C catalyst, is summarized in Table 3. For the Sb/C catalyst, increasing the antimony concentration from .0011 to .0017 (mol. SbCl₅/g) increased conversion from 78.7% to 99.6%. For the Ti/C catalyst, increasing concentration from .0017 to .0034 (mol. TiCl₄/g) increased conversion from 52% to 97.4%. These data suggest that increasing metal concentration on the support increases conversion for both antimony and titanium catalysts.

Example 4: Effect of HC1 and 141b on the performance of Sb/C catalyst.

The .0017 mol. Sb/g catalyst was evaluated between 70 and 100°C under a variety of processing conditions. At 70°C, 12.7 sec. contact time, 1.34 m.r. of HF/142b, conversion was 89.5%. When the temperature was increased to 100°C, conversion increased to 99.9%. Upon adding 141b, and increasing the HF feed m.r. (HF/2x141b+142b) to 1.73, using a contact time of 5.7 seconds, conversion was still 99.9%, which means that the catalyst was highly active to convert both 141b and 142b to the desired product, 143a. With HCl (13.3 mole % from the total feed) and a contact time of 4.9 seconds, conversion was approximately 96% (no co-product formed). When HC1 flow stopped, conversion again increased to 99.9%. Thus, the slight negative effect of HC1 on the catalyst performance is a reversible physical effect and causes no permanent damage to the chemical composition of the catalyst. A summary of these results is shown in Table 4. The presence of high levels of HC1 did not alter the overall conversion of the catalyst, so it is possible to feed impure 142b containing 141b and HC1 in the process of the present invention.

**Table 1**

| Summary of the catalyst Ti/C evaluation and the other two catalysts Sn/C and Sb/C. | | | | | | |
|---|---|---|---|---|---|---|
| Catalyst¹ | T°C | 142b x 10⁴ mole/min. | HF x 10⁴ mole/min. | Contact Time Seconds | % Conv. | % 143a |
| None | 140 | 8.9 | 26.7 | 47 | 0 | 0 |
| Sn/C | 140 | 8.9 | 26.7 | 47 | 91.1 | 100 |
| Sb/C | 140 | 8.9 | 26.7 | 47 | 100 | 100 |
| Ti/C | 140 | 8.9 | 26.7 | 47 | 84.7 | 100 |
| Ti/C | 160 | 8.9 | 26.7 | 54 | 99.2 | 100 |

| | | | | | | |
|---|---|---|---|---|---|---|
| 1. Metal concentration is .0017 mol./g. of the catalyst. | | | | | | |
| Activated carbon used is Calgon CPG. | | | | | | |

**Table 2**

| Results from Sb/C Catalyst at 100°C | | | |
|---|---|---|---|
| Time (Seconds) | m.r HF/142b | % Conversion | 143a % |
| 32 | 2.00 | 99.96 | 100 |
| 28 | 1.40 | 99.97 | 100 |
| 26 | 1.22 | 99.97 | 100 |
| 26 | 1.15 | 99.94 | 100 |
| 15 | 1.10 | 99.60 | 100 |

**Table 3**

| Effect of catalyst concentration on conversion of the catalyst. | | | | | |
|---|---|---|---|---|---|
| Catalyst mol./g. cat. | T°C | m.r HF/142b | Contact Time Seconds | % Conversion | % 143a Selectivity |
| .0011 Sb/C | 100 | 1.4 | 12 | 78.7 | 100 |
| .0017 Sb/C | 100 | 1.1 | 15 | 99.6 | 100 |
| .0017 Ti/C | 140 | 1.3 | 13.9 | 52 | 100 |
| .0034 Ti/C | 140 | 1.3 | 10.1 | 97.4 | 99.9 |

**Table 4**

| Summary of the effect of HCl and 141(b) on the performance of Sb/C catalyst at 1 atmosphere | | | | | | | | |
|---|---|---|---|---|---|---|---|---|
| T°C | 142b x 10³ mol/m | 141b x 10³ mol/m | HCl x 10³ mol/m | HF/(2x141b + 142b) | Cont. Time Sec. | % Conv. | % Select. | Hours |
| 70 | 1.9 | 0 | 0 | 1.34 | 12.7 | 89.5 | 100 | 9-109.2 |
| 100 | 1.9 | 0 | 0 | 1.34 | 11.7 | 99.9 | 100 | 113-175 |
| 100 | .94 | .7 | 0 | 1.73 | 5.7 | 99.9 | 100 | 177-243 |
| 100 | .94 | .7 | .6 | 1.73 | 4.9 | 96.0 | 100 | 347-420 |
| 100 | .94 | .7 | 0 | 1.73 | 5.7 | 99.9 | 100 | 431-489 |

While the invention has been described herein with reference to specific embodiments, it is not limited thereto. Rather it should be recognized that this invention may be practiced as outline above within the spirit and scope of the appended claims, with such variants and modifications as may be made by those skilled in this art.

## Claims

1. A process for manufacturing 1,1,1-trifluoroethane (143a), which comprises contacting 1,1-difluoro-1-chloroethane (142b) with HF in the gas phase, in the presence of a catalyst selected from the SbV, TiIV and SnIV salts.

2. The process of claim 1, wherein the catalyst is unsupported.

3. The process of claim 1, wherein the catalyst is supported.

4. The process of claim 1, wherein the catalyst is selected from SbCl₅, TiCl₄ and SnCl₄.

5. The process of claim 4, wherein the catalyst is SbCl₅.

6. The process of claim 5, wherein there is no co-feeding of chlorine gas or other oxidizer.

7. The process of claim 1 which is performed without co-feeding air or other oxygen containing gas.

8. The process of claim 1, which results in the formation of less than 10 ppm of olefin byproduct.

9. The process of claim 1, wherein 143a is manufactured with a selectivity of at least 98%.

10. The process of claim 1, wherein the reaction is conducted at a temperature of from 30°C to 250°C.

11. The process of claim 10, wherein the reaction temperature is from 50°C to 160°C.

12. The process of claim 1, wherein the molar ratio of HF to 1,1-difluoro-1-chloroethane is from 1:1 to 20:1.

13. The process of claim 12, wherein the molar ratio is from 1:1 to 10:1.

14. The process of claim 12, wherein the molar ratio is from 2:1 to 5:1.

15. The process of claim 1, wherein the reaction is conducted at a pressure between atmospheric and 200 psig.

16. The process of claim 15, wherein the pressure is between atmospheric and 150 psig.

17. The process of claim 10, wherein the contact time is from 1 to 100 seconds.

18. The process of claim 17, wherein the contact time is from about 1 to 60 seconds.

19. The process of claim 17, wherein the contact time is from about 5 to 50 seconds.

20. The process of claim 17, wherein the contact time is from about 5 to 15 seconds.

21. The process of claim 1, which is performed in the presence of an impurity selected from 1,1-dichloro-1-fluoroethane (141b) and HCl.

22. The process of claim 1, which is performed in the absence of HCl and 141b.
